# EUROPEAN PATENT SPECIFICATION

(11) **EP 3 290 401 B1**
(45) Date of publication and mention of the grant of the patent: **23.09.2020**
(21) Application number: 16001896.6
(22) Date of filing: 30.08.2016
(51) Int. Cl.: C07C 209/26, C07C 211/10

(54) **N-SUBSTITUTED ACYCLIC ETHYLENE DIAMINES**
N-SUBSTITUIERTE ACYCLISCHE ETHYLENDIAMINE
DIAMINES D'ÉTHYLÈNE ACYCLIQUE N-SUBSTITUÉS

(43) Date of publication of application: 07.03.2018
(73) Proprietor: Taminco bvba, 9000 Gent (BE)
(72) Inventor: Pelckmans, Michiel Jules Y, 3001 Heverlee (BE); VAN Waes, Frederik, 9000 Gent (BE); Sels, Bert Frans, 3001 Heverlee (BE); Moonen, Kristof, 9820 Merelbeke (BE)
(74) Representative: Ricker, Mathias

(56) References cited:
- GB-A- 844 448
- US-A- 2 016 962

## Description

### FIELD OF THE INVENTION

The invention relates to two alternative methods A and B of manufacturing N-substituted acyclic ethylene diamines and the use of the thus manufactured N-substituted acyclic ethylene diamines.

### BACKGROUND OF THE INVENTION

*N*-substituted acyclic ethylene diamines have all the following C2-structure in common, wherein R₁, R₂, R₃ and R₄ can be identical or different from each other:

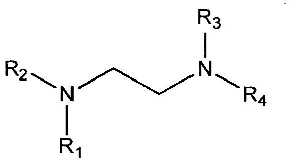

Industrially, e.g. ethylene diamine, one representative of the group of acyclic ethylene diamines, wherein R₁, R₂, R₃ and R₄ each signify hydrogen, is produced by reacting ammonia with 1,2-dichloroethane, ethylene glycol or monoethanol amine. Due to the generally higher activity of alkylamines compared to ammonia, ethylene diamine is not the only product formed in this process. Consecutive reactions also take place yielding diethylene triamine, triethylene tetramine, etc. but also heterocyclic compounds like piperazine as a consequence of an intramolecular reaction. Further N-alkylation can take place in a separate alkylation step (e.g. by reductive amination) or can be achieved by replacing ammonia with primary or secondary alkyl amines in the reaction with 1,2-dichloroethane, ethylene glycol or monoethanol amine.

The educts used for manufacturing ethylene diamine like ethylene glycol, monoethanolamine and 1,2-dichloroethane, however, are typically obtained through functionalization of a C2 petrochemical feedstock, in particular ethylene. In view of the fact that petrochemical raw materials will become more and more difficult to obtain in the future, it is therefore desirable to discover new, raw materials preferably from renewable sources for manufacturing *N*-substituted acyclic ethylene diamines.

In US 2,016,962 it is described that glucamines can be obtained in an reductive amination process using e.g ammonia and renewable sources like carbohydrates, in particular sugars as educts. In GB 844,448 and GB 449,474 it is described that i.a. N-substituted acyclic propylene diamines containing a C3-chain can be obtained if carbohydrates like sucrose are used as educts in said reductive aminolysis process.

### OBJECTS OF THE INVENTION

It was therefore an object of the present application to provide methods for the manufacturing of *N*-substituted acyclic ethylene diamines using compounds originating from renewable biomass feedstock as starting materials.

### SUMMARY OF THE INVENTION

This object is solved by two alternative methods, method A and method B, for producing at least one *N*-substituted acyclic ethylenediamine:
Method A for producing at least one *N*-substituted acyclic ethylenediamine, comprising reacting at least one reducing sugar, hydrogen and at least one primary amine NH₂R or at least one secondary amine NHR'R" or a mixture of at least one primary amine NH₂R and at least one secondary amine NHR'R" in the presence of a supported hydrogenation catalyst at a reaction temperature of 50°C to 200 °C, and at a reaction pressure being superatmospheric pressure, and wherein the molar ratio of the at least one primary amine and/or secondary amine : reducing sugar is at least 6: 1.
Method B for producing at least one N-substituted acyclic ethylene diamine, comprising the steps of reacting at least one reducing sugar and at least one primary amine NH₂R or at least one secondary amine NHR'R" or a mixture of at least one primary amine NH₂R and NHR'R" in a first step, and reacting the reaction mixture obtained in the first step with hydrogen in the presence of a supported hydrogenation catalyst in a second step.

In particular, the methods according to the invention comprise the provision that no unsupported hydrogenation catalyst is used, in particular no spongy hydrogenation catalyst or no hydrogenation catalyst of the Raney-type.

In particular, the methods according to the invention comprise that the supported hydrogenation catalyst comprises a metal, in particular copper, nickel, cobalt, iron, ruthenium, platinum, palladium, and two or more thereof.

In particular, the methods according to the invention comprise that the supported hydrogenation catalyst comprises a support substrate for the catalytic active metal selected from carbon, a polymer or a metal oxide.

In particular, the methods according to the invention comprise that the residue R of the at least one primary amine or the residues R' and R" of the at least one secondary amine are the same or different and each independently selected from hydrogen, linear or branched alkyl groups, linear or branched hydroxyalkyl groups, cyclic alkyl groups, which may be substituted and aromatic groups, which may be substituted.

In particular, the methods according to the invention comprise the provision that no ammonia (NH₃) is used as educt for the production of N-substituted acyclic ethylene diamines.

In particular, the methods according to the invention comprise that the at least one reducing sugar is selected from a monosaccharide, a disaccharide, an oligosaccharide or a polysaccharide, and a mixture of two or more thereof.

In particular, the methods according to the invention comprise that at most 10 C% of heterocyclic by-products are formed based on the total amount of reducing sugar used as educt.

In particular, the methods according to the invention comprise that the at least one primary amine and/or the at least one secondary amine can be used in form of a solution, wherein the at least one primary amine and/or the at least one secondary amine is dissolved in an aprotic solvent or a protic solvent, in particular water, or methanol, or DMF, or dimethylacetamide.

In particular, the methods according to the invention comprise that at least one primary amine and/or the at least one secondary amine is used in gaseous form.

In particular, the methods according to the invention comprise that the reducing sugar is used in solid form or in the form of a solution, wherein the reducing sugar is dissolved in water, alcohols, ethers or dipolar aprotic solvents.

In particular, the methods according to the invention comprise that the method is performed in a batchwise manner, or a fed-batch manner or in a continuous manner.

One further aspect of the invention comprise the use of the at least one N-substituted acyclic ethylenediamine obtainable according to the methods according to the invention as building block for surfactants and fabric softener, as epoxy curing agent, as catalyst for manufacturing polyurethane, as ligand for metals.

In particular, the use of the at least one *N*-substituted acyclic ethylenediamine obtainable according to the methods according to the invention comprises that the at least one *N*-substituted acyclic ethylenediamine obtainable according to the methods according to the invention are used without any further purification, or wherein the at least one *N*-substituted acyclic ethylenediamine obtained according to the methods according to the invention are used after purification.

### DETAILED DESCRIPTION OF THE INVENTION

Claimed herein are two alternative methods for producing at least one N-substituted acyclic ethylene diamine:
Method A according to the invention comprises:
   - reacting at least one reducing sugar, hydrogen and at least one primary amine NH₂R or at least one secondary amine NHR'R" or a mixture of at least one primary amine NH₂R and at least one secondary amine NHR'R"
   - in the presence of a supported hydrogenation catalyst
   - at a reaction temperature of 50°C to 200 °C, and
   - at a reaction pressure being superatmospheric pressure, and wherein
   - the molar ratio of the at least one primary amine and/or secondary amine : reducing sugar is at least 6:1.
Method B according to the invention comprises the steps:
   1. reacting at least one reducing sugar and at least one primary amine NH₂R or at least one secondary amine NHR'R" or a mixture of at least one primary amine NH₂R and one secondary amine NHR'R" in a first step, and
   2. reacting the reaction mixture obtained in step 1) with hydrogen in the presence of a supported hydrogenation catalyst in a second step.

Methods A and B according to the invention for producing at least one *N-*substituted acyclic ethylenediamine, have the advantage that N-substituted acyclic ethylene diamines can be produced in high yields.

Without wanting to be bound to any theory, the inventors assume that the conditions provided by the alkyl amine environment, allow retro-aldol type fragmentation to occur at the same temperature range as is typically needed for the hydrogenation step. As such, both reaction steps can advantageously be effectuated in one reactor chamber, in the most optimal set of conditions for each step, leading to minimal side reactions as for example cyclization reactions, leading to piperazine derivatives.

In one embodiment, method A according to the invention for producing at least one *N*-substituted acyclic ethylenediamine, consists only of reacting at least one reducing sugar, hydrogen and at least one primary amine NH₂R or at least one secondary amine NHR'R" or a mixture of at least one primary amine and at least one secondary amine in the presence of a supported hydrogenation catalyst at a temperature of between 50°C to 200 °C, and at superatmospheric pressure, and wherein the molar ratio of the at least one primary amine and/or secondary amine : reducing sugar is at least 4:1.

In one embodiment, method B according to the invention for producing at least one N-substituted aryclic ethylene diamine consists only of the steps
1. reacting at least one reducing sugar and at least one primary amine NH₂R or at least one secondary amine NHR'R" or a mixture of at least one primary amine NH₂R and at least one secondary amine NHR'R" in a first step, and
2. reacting the reaction mixture obtained in step 1) with hydrogen in the presence of a supported hydrogenation catalyst in a second step.

Preferably, *N*-substituted acyclic ethylene diamines may be produced by the methods A and B according to the invention in yields of at least 30 C%, preferably at least 35 C%, preferably at least 40 C%, preferably at least 45 C%, preferably at least 50 C%, preferably at least 55 C%, more preferably at least 60 C%, more preferably at least 65 C%, more preferably at least 70 C%, more preferably at least 75 C%, more preferably at least 80 C%, more preferably at least 85 C%, more preferably at least 90 C% and more based on the total amount of reducing sugar used as educt.

Yields are expressed in carbon percent (C%) unless otherwise stated. The term *"carbon percent yield (C%)*" within the context of the present application means the yield of (by)product(s) which can be calculated by dividing the number of moles of carbon derived from the residue of the reducing sugar molecule in the (by)product in the reaction mixture, by the total number of moles of carbon supplied to the reaction via the reducing sugar educt. The carbon atoms that originate from the at least one primary and/or secondary amine are not taken into account for the yield determination.

The term *"N-substituted acyclic ethylene diamine"* within the context of the present application means compounds according to the following formula: 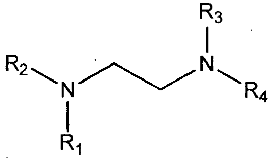 wherein the residues R¹, R², R³ and R⁴ can be identical or different from each other. In particular, the residues R¹, R², R³ and R⁴ are determined by the residues R, R', R" of the at least one primary amine NH₂R, respectively the at least one secondary amine NHR'R" or the mixture of the at least one primary amine or the at least one secondary amine used as educts in the methods according to the invention. Consequently, the residues R¹, R², R³ and R⁴ are each independently selected from hydrogen, linear alkyl groups, branched alkyl groups, linear hydroxy alkyl groups, branched hydroxy alkyl groups, cyclic alkyl groups, which may be substituted and aromatic groups, which may be substituted. Further, the residues R¹, R², R³ and R⁴ can form together with the nitrogen atom to which the residues R¹, R², R³ and R⁴ are attached a heterocyclic alkyl group or a heterocyclic aromatic group, like piperidine, pyrrolidine, morpholine, or piperazine, which may be substituted on the carbon ring.

The residues R¹, R², R³ and R⁴ can be each independently selected from hydrogen, methyl, ethyl, n-propyl, iso-propyl, n-butyl, s-butyl, t-butyl, iso-butyl, n-pentyl, sec-pentyl, iso-pentyl, n-hexyl, sec-hexyl, iso-hexyl, cyclohexyl, hydroxy ethyl, hydroxyl propyl, hydroxy-iso-propyl, 1,2-dihydroxy propyl, 1-hydroxy butyl, 2- hydroxy butyl, 3-hydroxy butyl, 4- hydroxy butyl, 1-hydroxy pentyl, 2- hydroxy pentyl, 3- hydroxy pentyl, 4-hydroxy pentyl, 5- hydroxy pentyl, 1-hydroxy hexyl, 2-hydroxy hexyl, 3-hydroxy hexyl, 4-hydroxy hexyl, 5-hydroxy hexyl, 6-hydroxy hexyl, toluyl, phenyl, 2-ethyl hexyl.

In particular, *N*-substituted acyclic ethylene diamine products obtained by the methods according to the invention can be, but are not limited to N,N'-dimethyl ethylene diamine (DMEDA), *N,N,N',N'*-tetra methyl ethylene diamine (TMEDA), *N,N'*-di-t-butyl ethylene diamine (DtBEDA), *N,N'*-bis(2-hydroxyethyl)ethylene diamine (BHEEDA), N,N'-bis(2-hydroxyethyl)-N,N'-dimethyl ethylenediamine (BHEDMEDA).

Further, the methods according to the invention have the advantage that the formation of heterocyclic by-products like *N*-alkylated piperazines, *C*-alkylated piperazines or unsubstituted piperazines can be reduced, preferably minimized.

Preferably at most 10 C%, further preferably at most 9 C%, further preferably at most 8 C%, further preferably at most 7 C%, further preferably at most 6 C%, further preferably at most 5 C%, further preferably at most 4 C%, more preferred at most 3 C%, even more preferred at most 2 C%, even more preferred at most 1 C% of heterocyclic by-products are formed based on the total amount of reducing sugar used as educt.

Such heterocyclic by-products formed may be cyclic ethylene diamine derivatives like *N*-alkylated piperazines, *C*-alkylated piperazines, N-alkylated C-alkylated piperazines or unsubstituted piperazine.

Further, the methods according to the invention have the advantage that the formation of compounds containing a C3-fragment, i.e. a chain consisting of 3 carbon atoms can be reduced, preferably minimized. Examples for compounds containing a C3-fragment are for example *N,N,N',N'*-tetra alkyl propylene diamine (e.g. *N,N,N',N'-*tetramethyl propylene diamine: TMPDA), *N,N*-dialkylamino-2-propanol (e.g. *N,N-*dimethylamino-2-propanol: DMA-2-propOH) or *N,N*-dialkylamino-2-propanone (e.g. *N,N-*dimethylamino-2-propanone: DMA-2-propanone); *N,N'*-dialkyl propylene diamine, N-alkylamino-2-propanol or N-alkylamino-2-propanone.

Preferably at most 15 C%, further preferably at most 12 C%, at most 10 C%, further preferably at most 9 C%, further preferably at most 8 C%, further preferably at most 7 C%, further preferably at most 6 C%, further preferably at most 5 C%, further preferably at most 4 C%, more preferred at most 3 C%, even more preferred at most 2 C%, even more preferred at most 1 C% of compounds containing a C3-fragment are formed based on the total amount of reducing sugar used as educt.

Further, the methods according to the invention have the advantage that compounds, in particular reducing sugars originating from renewable feedstock can be used as educts.

The term *"reducing sugar(s)"* within the context of the present application means sugars having at least one free aldehyde group (aldose) or a free, tautomerizable ketone group (ketose). Also derivatives of reducing sugars are encompassed by the term reducing sugar(s) within the context of the present application. In particular, reducing sugars are selected from monosaccharides, disaccharides, oligosaccharides or polysaccharides or mixtures thereof.

The term *"monosaccharide(s)"* within the context of the present application is defined as follows:

A monosaccharide is a simple sugar with three, four, five or six carbon atoms, in which the carbon atoms are connected in a single, uninterrupted carbon chain. Monosaccharides can be converted from the acyclic, open-chain form into the cyclic form and vice versa. For conducting the methods according to the invention it is not decisive which form is predominant. Examples of monosaccharides suitable for being used as reducing sugar in the methods according to the invention are galactose, glucose, glyceraldehyde, fructose, ribose, xylose, mannose, arabinose, dihydroxyacetone, erythrose, or erythrulose. Glycolaldehyde is excluded from the list of suitable monosaccharides.

In one embodiment of the methods according to the invention glucose is the preferred monosaccharide.

The term *"disaccharide(s)"* within the context of the present application is defined as follows:

Disaccharides consist of two identical or two different monosaccharides interlinked with a C-O-C chemical bond. Disaccharides can be divided into the group having reducing properties and thus being usable as reducing sugars within the context of the present application and further into the group of having no reducing properties (also referred to herein as *"non-reducing sugars"*). Generally disaccharides having reducing properties can be distinguished from disaccharides having no reducing properties in that one monosaccharide is present having a free hemiacetal unit at the anomeric carbon atom and thus allows for a conversion of one monosaccharide moiety into the open-chain form having a free carbonyl group. In contrast thereto, in non-reducing sugars like sucrose or trehalose the anomeric carbon atoms are blocked due to glycosidic bonds. Consequently, no conversion into the open-chain form can take place. Examples of disaccharides acting as reducing sugars are lactose, cellobiose, melibiose, xylobiose, isomaltose, mannobiose and maltose.

In one embodiment of the methods according to the invention maltose is the preferred disaccharide.

The terms *"oligosaccharide(s)"* and *"polysaccharide(s)"* within the context of the present application are defined as follows:

Oligo- and polysaccharides may be used as such in the methods according to the invention, or maybe cleaved prior to be used in the methods according to the invention in order to obtain the monosaccharide moieties, respectively disaccharide moieties. For example, disaccharide cellobiose is a hydrolysis product of polysaccharide cellulose, or disaccharide maltose is a hydrolysis product of polysaccharide starch. Both, oligo- and polysaccharides, are built-up by identical or different monosaccharide moieties. The difference between oligo- and polysaccharides can be seen in the number of monosaccharide moieties. Oligosaccharides can contain up to 10 monosaccharide moieties. Polysaccharides usually contain numerous, e.g. several hundred of monosaccharide moieties. Oligo- and polysaccharides can be derived from renewable feedstock biomass like plants. Further, also oligosaccharides can be derived from polysaccharides e.g. by microbial breakdown. Examples of oligosaccharides which can be used as educt in the methods according to the invention are maltodextrin, maltotriose, or cello-oligomers, like cellotriose or cellotetrose, and examples of polysaccharides which can be used as educt in the methods according to the invention are starch, cellulose, or partially hydrolyzed polysaccharides like partially hydrolyzed starch or partially hydrolyzed cellulose or maltodextrins. A partial hydrolysis of polysaccharides can be achieved by enzymatic or chemical catalysis.

In the methods according to the invention at least one reducing sugar can be used as educt selected from monosaccharide, disaccharide, oligosaccharide or polysaccharide or mixtures thereof.

In one embodiment of the methods according to the invention one reducing sugar can be used as educt selected from monosaccharide, disaccharide, oligosaccharide or polysaccharide.

In one embodiment of the methods according to the invention two or more, e.g. three or four reducing sugars can be used as educts selected from monosaccharide, disaccharide, oligosaccharide or polysaccharide or mixtures thereof.

The at least one reducing sugar can be used in solid form. The at least one reducing sugar can be used in form of a solution, wherein the at least one reducing sugar is dissolved in at least one solvent. Suitable solvents are for example water; protic solvents like alcohols; ethers; dipolar aprotic solvents; or mixtures thereof.

In one embodiment of the methods according to the invention alcohols are the preferred solvents.

The term *"primary amine"* within the context of the present application means a compound which is capable of acting as an aminating agent and having the formula NH₂R, wherein the residue R is selected from linear or branched alkyl groups,linear or branched alkanol groups, cyclic alkyl groups which may be substituted and aromatic groups, which may be substituted.

In particular, residue R can be selected from methyl, ethyl, hydroxy ethyl, hydroxyl propyl, hydroxy-iso-propyl, 1,2-dihydroxy propyl, 1-hydroxy butyl, 2- hydroxy butyl, 3-hydroxy butyl, 4-hydroxy butyl, 1-hydroxy pentyl, 2-hydroxy pentyl, 3- hydroxy pentyl, 4-hydroxy pentyl, 5- hydroxy pentyl, 1-hydroxy hexyl, 2-hydroxy hexyl, 3-hydroxy hexyl, 4-hydroxy hexyl, 5-hydroxy hexyl, 6-hydroxy hexyl, toluyl or phenyl.

Examples of primary amines suitable to be used in the methods according to the invention can be selected from methylamine, ethylamine, 2-ethanolamine, n-propylamine, isopropylamine, 3-propanolamine, isopropanolamine, 3-aminopropane-1,2-diol, n-butylamine, s-butylamine, isobutylamine, 2-butanolamine, 3-butanolamine, 4-butanolamine, n-pentylamine, s-pentylamine, isopentylamine, 2-pentanolamine, 3-pentanolamine, 4-pentanolamine, 5-pentanolamine, n-hexylamine, s-hexylamine, isohexylamine, cyclohexylamine, 2-hexanolamine, 3-hexanolamine, 4-hexanolamine, 5-hexanolamine, 6-hexanolamine, toluidine or aniline.

The term *"secondary amine"* within the context of the present application means a compound which is capable of acting as an aminating agent and having the formula NHR'R", wherein the residues R' and R" can be identical or different from each other and can each be independently selected from linear or branched alkyl groups, linear or branched alkanol groups, cyclic alkyl groups which may be substituted and aromatic groups which may be substituted. Further, the residues R' and R" can form together with the nitrogen atom to which the residues R' and R" are attached a heterocyclic alkyl group or a heterocyclic aromatic group, like piperidine, pyrrolidine, morpholine, piperazine.

In particular, residues R' and R" can be independently from each other selected from methyl, ethyl, ethanol, n-propyl, iso-propyl, propanol, iso-propanol, propane-1,2-diol, n-butyl, sec-butyl, iso-butyl, 2-butanol, 3-butanol, 4-butanol, n-pentyl, sec-pentyl, iso-pentyl, 2-pentanol, 3-pentanol, 4-pentanol, 5-pentanol, n-hexyl, sec-hexyl, iso-hexyl, cyclo-hexyl, 2-hexanol, 3-hexanol, 4-hexanol, 5-hexanol, 6-hexanol, toluyl or phenyl.

Examples of secondary amines suitable to be used in the methods according to the invention can be selected from dimethylamine, methylethylamine, diethylamine, N-methyl-2-ethanolamine, N-ethyl-2-ethanolamine, N-propyl-2-ethanolamine, N-butyl-2-ethanolamine, N-pentyl-2-ethanolamine, N-hexyl-2-ethanolamine, di-2-ethanolamine, din-propylamine, di-s-propylamine, diisopropylamine, isopropylmethylamine, isopropylethylamine, N-methyl-2-propanolamine, N-methyl 3-propanolamine, dipropanolamine, di-n-butylamine, di-s-butylamine, diisobutylamine, N-methyl-2-butanolamine, N-methyl-3-butanolamine, N-methyl-4-butanolamine, N-ethyl-2-butanolamine, N-ethyl-3-butanolamine, N-ethyl-4-butanolamine, N-propyl-2-butanolamine, N-propyl-3-butanolamine, N-propyl-4-butanolamine, N-butyl-2-butanolamine, N-butyl-3-butanolamine, N-butyl-4-butanolamine, di-2-butanolamine, di-3-butanolamine, di-4-butanolamine, di-n-pentylamine, di-s-pentylamine, diisopentylamine, N-methyl-5-pentanolamine, N-ethyl-5-pentanolamine, N-propyl-5-pentanolamine, N-butyl-5-pentanolamine, N-pentyl-5-pentanolamine, di-2-pentanolamine, di-3-pentanolamine, di-4-pentanolamine, di-5-pentanolamine, di-n-hexylamine, di-s-hexylamine, diisohexylamine, N-methyl-2-hexanolamine, N-methyl-3-hexanolamine, N-methyl-4-hexanolamine, N-methyl-5-hexanolamine, N-methyl-6-hexanolamine, N-ethyl-2-hexanolamine, N-ethyl-3-hexanolamine, N-ethyl-4-hexanolamine, N-ethyl-5-hexanolamine, N-ethyl-6-hexanolamine, N-propyl-2-hexanolamine, N-propyl-3-hexanolamine, N-propyl-4-hexanolamine, N-propyl-5-hexanolamine, N-propyl-6-hexanolamine, N-butyl-2-hexanolamine, N-butyl-3-hexanolamine, N-butyl-4-hexanolamine, N-butyl-5-hexanolamine, N-butyl-6-hexanolamine, N-pentyl-2-hexanolamine, N-pentyl-3-hexanolamine, N-pentyl-4-hexanolamine, N-pentyl-5-hexanolamine, N-pentyl-6-hexanolamine, N-hexyl-6-hexanolamine, di-2-hexanolamine, di-3-hexanolamine, di-4-hexanolamine, di-5-hexanolamine, di-6-hexanolamine, piperidine, pyrrolidine, piperazine or morpholine.

In general, at least one primary amine or at least one secondary amine or a mixture of at least one primary amine and at least one secondary amine can be used in the method according to the invention.

In one embodiment of the methods according to the invention only one primary amine is reacted with the reducing sugar and hydrogen.

In one embodiment of the methods according to the invention two or more, e.g. three or four primary amines are reacted with the reducing sugar and hydrogen.

In one embodiment of the methods according to the invention only one secondary amine is reacted with the reducing sugar and hydrogen.

In one embodiment of the methods according to the invention two or more, e.g. three or four secondary amines are reacted with the reducing sugar and hydrogen.

In case a mixture of at least one primary amine and at least one secondary amine is used in the methods according to the invention, no restriction with respect to the molar ratios between the at least one primary amine and the at least one secondary amine need to be considered. It is, however, preferable to use the at least one primary amine in slight excess in order to compensate the higher reactivity of the at least one secondary amine

In one embodiment of the methods according to the invention one primary amine and one secondary amine are reacted with the reducing sugar and hydrogen.

In one embodiment of the methods according to the invention one primary amine and two or more, e.g. three or four secondary amines are reacted with the reducing sugar and hydrogen.

In one embodiment of the methods according to the invention two or more, e.g. three or four primary amines and one secondary amine are reacted with the reducing sugar and hydrogen.

In one embodiment of the methods according to the invention, two or more, e.g. three or four primary amines and two or more, e.g. three or four secondary amines are reacted with the reducing sugar and hydrogen.

However, in any case, no ammonia (NH₃) is used as educt for the production of N-substituted acyclic ethylene diamines.

The at least one primary amine and/or the at least one secondary amine can be used in gaseous form. The at least one primary amine and/or secondary amine can be also used in liquid form. The at least one primary amine and/or one secondary amine can be also used in form of a solution, wherein the at least one primary amine and/or the at least one secondary amine is dissolved in protic solvent or non-protic solvent or protic solvent mixture or non-protic solvent mixture. Suitable solvents are e.g. water, methanol, ethanol, iso-propanol, dimethylaminoethanol, DMF, NMP or dimethylacetamide. Preferred solvents are polar protic solvents such as methanol, ethanol, methylaminoethanol or dimethylaminoethanol. The at least one primary amine and/or the at least one secondary amine can be used in solid form, e.g. in form of a salt..

Preferably, in the methods according to the invention the amount of water by weight present in the reaction mixture at the beginning of the reaction is at most 1 time higher, preferably at most 2 times higher, preferably at most 3 times higher, preferably at most 4 times higher, preferably at most 5 times higher, preferably at most 6 times higher than the amount of reducing sugar educt by weight.

In the methods according to the invention a molar ratio of the at least one primary amine and/or secondary amine: at least one reducing sugar is at least 6:1, preferably at least 7:1, preferably at least 8:1, preferably at least 9:1, preferably at least 10:1, preferably at least 11:1, and more preferably at least 12 :1, preferably at least 13:1.

One further advantage of the methods A and B according to the invention is that the use of a supported hydrogenation catalyst results in the formation of *N*-substituted acyclic ethylene diamines in high yields and high purity. In particular, the formation of by-products like heterocyclic compounds like piperazines or derivatives thereof, or compounds containing C3-fragments such as *N,N,N'N'*-tetramethyl propylenediamine (TMPDA); *N,N*-dimethylamino-2-propanol (DMA-2-PropOH); or *N,N*-dimethylamino-2-propanone (DMA-2-Propanone), or other compounds like DMAE can be minimized by using the supported hydrogenation catalyst in the methods according to the invention. The term *"supported hydrogenation catalyst"* within the context of the present application is defined as follows:

Sometimes in literature supported hydrogenation catalysts are also called bifunctional hydrogenation catalysts due to the presence of an acid/base functionality provided by the support part of the catalyst and the property to activate hydrogen due to the metal part of the catalyst. In any case, the respective catalyst possesses hydrogenation capacity towards a substrate. In the methods according to the invention, the supported hydrogenation catalyst used shows hydrogenation capacity towards the at least one reducing sugar or reducing sugar - amine adducts and fragments thereof.

The supported hydrogenation catalyst used in the methods according to the invention has to be distinguished from unsupported hydrogenation catalysts (also referred to in the literature as monofunctional hydrogenation catalysts), in particular from hydrogenation catalysts of the Raney-type, e.g. Raney-nickel or spongy hydrogenation catalysts, e.g. spongy nickel. Hydrogenation catalysts of the Raney-type or spongy hydrogenation catalysts are not suitable to be used in the methods according to the invention since the use of the same results in worse yields of N-substituted acyclic ethylene diamines and consequently also in a worse purity since by-products are produced in a higher quantity.

Supported hydrogenation catalysts which are suitable to be used in the methods according to the invention comprise, respectively consist of a metal part and a support part.

The metal part of the supported hydrogenation catalyst consists of at least one metal. This at least one metal has the property to activate hydrogen in order to facilitate the hydrogenation of a substrate like e.g. reducing sugar, and therefore is present, at least to some extent, in the zero ("0") oxidation state under the operating conditions and can be regarded as catalytic active metal center.

The at least one metal is preferably selected from copper, nickel, cobalt, iron, ruthenium, platinum, palladium, or two or more thereof. In case two or more metals are present in the supported hydrogenation catalyst, these two or more metals can be present in form of an alloy; these two or more metals can exist next to each other in separate so-called crystallites without forming an alloy or the like; or these two or more metals can be present in metal crystallites consisting of zones of each individual metal without forming an alloy or the like (e.g. the metal crystallites can consist of layers of the individual metals).

The support part of the hydrogenation catalyst comprises or consists of at least one support substrate for the at least one catalytic active metal center of the metal part of the supported hydrogenation catalyst. The at least one metal is located on the surface of the support substrate. The support substrate preferably embeds the at least one metal.

The at least one support substrate can be carbon, a polymer or a (mixed) metal oxide.

A carbon support substrate can be an activated carbon (AC), obtained from a suitable natural material such as peat, wood, coconut husk, nut shells, lignite etc; a modified activated carbon (e.g. by oxidation steaming or sulfonation); graphite or synthetic carbon nanotubes.

A polymer support substrate can be poly (acrylic acid), polystyrene, poly (styrene-co-divinylbenzene), or polyamides.

A metal oxide support substrate can be silica (SiO₂); alumina (Al₂O₃); or silica-alumina mixture (SiO₂/Al₂O₃), either in an amorphous form or in a crystalline form (e.g. zeolites); TiO₂; ZrO₂. Further, the metal oxide support can also be the oxide of one of the catalytic active metals as defined above. The catalytic active metal center of the supported hydrogenation catalyst can hence be generated by partial reduction of the metal oxide in the supported hydrogenation catalyst, leaving sufficient metal oxide support substrate to support the catalytic active metal center.

Examples of supported hydrogenation catalysts suitable to be used in the methods according to the invention can be selected from Ni-6458P (BASF), Ni-5249P (BASF), Ni-3354E (BASF), Ni/SiO₂-Al₂O₃ (65 wt% Ni, Sigma-Aldrich), Ni-2495P (BASF), KL6504K-P (71 wt% Ni, CRI), or Ru/C (5wt% Ru, Sigma-Aldrich).

In one embodiment, the supported hydrogenation catalyst consists of one metal and one support substrate. Such a supported hydrogenation catalyst can be selected from supported nickel hydrogenation catalysts or Ru/C catalysts.

In one embodiment, the supported hydrogenation catalyst consists of two or more metals and two or more support substrates.

The supported hydrogenation catalyst can be directly added to the reaction mixture in solid form, e.g. as a powder. In such a case, the supported hydrogenation catalyst is suspended in the liquid reaction medium by a suitable way of agitation, for instance an axial stirrer or a circulation pump. The supported hydrogenation catalyst can be separated from the reaction medium after the reaction has been completed e.g. by filtration, centrifugation or settlement. The amount of the supported catalyst added to the reaction mixture depends on the total amount of liquids present in the reaction mixture in order to maintain stirrability, respectively pumpability of the reaction mixture. Generally, the amount of supported catalyst can be up to 30 weight%, or up to 25 weight%, or up to 20 weight%, or up to 15 weight%, or up to 10 weight%, or up to 7 weight% or up to 5 weight%, or up to 3 weight%, or up to 1 weight%.

Alternatively, the supported hydrogenation catalyst can be fixed in the reactor while the reaction mixture, consisting of liquids and or gases, is circulated over the fixed catalyst bed. The fixed catalyst bed can for instance be supported into a tubular reactor and the reaction mixture is equally distributed over the cross section of the tube to assure optimal contact of the reaction medium and the catalyst particles. The reactor can be filled with either one bed of catalyst particles, but also consecutive beds of catalyst can be placed into the tubular reactor, optionally with liquid and/or gas distributers between some or all of the beds. To allow the supported hydrogenation catalyst to be packed in a bed and at the same time leaving sufficient freedom for the reaction mixture to pass, the supported hydrogenation catalyst may be in a powderous form. Preferably the supported hydrogenation catalyst is "formed" into a suitable catalyst particle. Such particle can be of cylindrical, spherical, bilobe, trilobe or any other suitable shape, but can also be in the shape of a monolith, allowing the reaction medium to flow past and through it. Forming of catalyst particles can be done by any method known by the person skilled in the art such as pelletizing, tableting, spray drying, extrusion, granulating, etc. The reaction mixture can move either from top to bottom over the catalyst bed, or from bottom to top. Gas and liquid streams can be either in the same direction (co-current) or in opposite direction (counter-current). Circulation can be in a way that the reaction mixture passes the fixed catalyst bed only once (once through) or that it is circulated multiple times over the fixed catalyst bed. Product withdrawal from the circulation stream can be either continuous or all at once at the end of the reaction after a predefined time of circulation.

The term *"reaction mixture"* within the context of the present application means a mixture of at least reducing sugar and primary and/or secondary amine.

The reaction mixture can further contain the at least one supported hydrogenation catalyst and/or additional solvent, like water, which was used for dissolving e.g. the at least one reducing sugar and/or the at least one primary amine and/or the at least one secondary amine and/or intermediate products of the reaction and/or end products of the reaction and/or byproducts of the reaction. In case of method B, the supported hydrogenation catalyst can be already present in the reaction mixture in the first step, or can be added to the reaction mixture at the beginning of the second step.

The reaction mixture can be either a homogeneous reaction mixture, e.g. a solution or a heterogeneous reaction mixture, e.g. a suspension or a dispersion. Further, the reaction mixture can also contain hydrogen, either in gaseous form or the hydrogen can be at least partly dissolved in e.g. the liquid components of the reaction mixture. In case of method A, the reaction mixture can contain hydrogen right from the beginning of the reaction. In case of method B, the reaction mixture contains hydrogen only after step 1 is completed; i.e. only as early as step 2 is conducted.

In one embodiment, the methods according to the invention are conducted without any additional solvent like water, alcohols or the like. In this embodiment, the at least one primary amine and/or the at least one secondary amine serve as reactant as well as solvent for dissolving the at least one reducing sugar.

The term *"hydrogen"* within the context of the present application means elementary hydrogen H₂. The hydrogen is used in the methods according to the invention in gaseous form.

One further advantage of the methods according to the invention is that the methods can be conducted under mild reaction temperature without negative influence on the yield and purity of *N*-substituted ethylene diamine(s) obtained as product(s).

The term *"reaction temperature"* within the context of the present application means the temperature to which the reaction mixture is heated (maybe also called "reaction temperature set point") and which is maintained until completion of the reaction. The reaction mixture can be heated up to the reaction temperature set point e.g. under stirring. During the heating phase, the temperature can overshoot the reaction temperature set point and then the temperature is lowered until the desired reaction temperature set point is achieved.

The reaction temperature, respectively the reaction temperature set point used in the methods according to the invention is at least 50°C, or at least 75°C, or at least 100°C, or at least 110°C, or at least 120°C, or at least 130°C, or at least 140°C, or at least 150°C, or at most 160°C, or at most 170°C, or at most 180°C, or at most 190°C, or at most 200°C. Preferably, the reaction temperature is at least 50°C and at most 200°C, preferably at least 100°C and at most 150°C, preferably 120°C.

The term *"reaction pressure"* within the context of the present application means the pressure which is used, respectively which is applied to the reaction mixture prior to start heating the reaction mixture to the reaction temperature. The temperature of the reaction mixture when the reaction pressure is applied can be between 10°C to 30°C, but is usually the same temperature as the ambient temperature. The temperature of the reaction mixture when the reaction pressure is applied can be also higher than ambient temperature if e.g. pre-heated educts or a pre-heated reaction mixture is used. The reaction pressure has to be distinguished from the pressure during the reaction. The reaction pressure can be e.g. applied to the reaction mixture by filling in gases like nitrogen or hydrogen into the reaction vessel until the desired reaction pressure is achieved.

The reaction pressure used in the methods according to the invention is superatmospheric pressure which means any pressure which is higher than the standard ambient pressure of 1 bar.

In particular, the reaction pressure used in the methods according to the invention is at least 10 bar, or at least 30 bar, or at least 50 bar, or at least 60 bar, or at least 70 bar, or at least 100 bar, or at most 110 bar, or at most 120 bar, or at most 130 bar, or at most 140 bar, or at most 150 bar, or at most 160 bar, or at most 170 bar, or at most 180 bar, or at most 190 bar, or at most 200 bar. Preferably, the reaction pressure is at least 10 bar and at most 200 bar, preferably at least 50 bar and at most 150 bar, preferably 75 bar. Also reaction pressures of above 200 bar are possible. The upper limit of the reaction pressure is determined by the equipment used.

The term *"pressure during the reaction"* within the context of the present application means the pressure which can be observed when heating of the reaction mixture is started, respectively if the temperature of the reaction mixture is further increased. When the reaction is performed in a closed vessel, the pressure increases due to thermal expansion of the compounds present in the reaction mixture during heating of the reaction mixture up to the reaction temperature set point. Thus, the pressure during the reaction is at least for a certain time higher than the reaction pressure prior to the start of heating the reaction mixture. The pressure during the reaction raises up to a maximum value. This maximum value of the pressure during the reaction depends on the set-up, geometry and filing degree of the reaction vessel.

Further, when heating is started, respectively if the temperature of the reaction mixture is further increased, the components within the reaction vessel start to react with each other. This means that hydrogen is consumed. In case of method B, hydrogen consumption takes place in step 2. Hydrogen consumption (and in some embodiments depending on the at least one primary and/or secondary amine used also the consumption of volatile primary and/or secondary amine), however, leads to a decrease of the pressure during the reaction. Thus, two contrary effects influence the pressure during the reaction at the same time until reaction is complete: 1. Thermal expansion of the components of the reaction mixture due to heating result in an increase of the pressure during the reaction and 2. Consumption of hydrogen (and in some embodiments depending on the at least one primary and/or secondary amine used also the consumption of volatile primary and/or secondary amine) due to reaction with the other components present in the reaction mixture lead to a decrease of the pressure during the reaction. When heating is started, respectively if the temperature of the reaction mixture is further increased, the thermal expansion is the predominating effect and therefore the pressure during the reaction increases up to a maximum value. Then, the consumption of hydrogen (and in some embodiments depending on the at least one primary and/or secondary amine used also the consumption of volatile primary and/or secondary amine) becomes the predominating effect and therefore, a decrease of the pressure during the reaction can be observed, although heating is not switched of, respectively is still ongoing.

If the pressure during the reaction starts to decrease, this shows that reaction takes place inside the reaction vessel and reducing sugar, primary amine and/or secondary amine and hydrogen are reacted at least partly to N-substituted ethylene diamines.

After a certain reaction time, the pressure during the reaction neither decreases any further, nor increases again; a constant value of the pressure during the reaction can be observed. If the pressure during the reaction does not change any more, this signals that the reaction is complete.

If now e.g. heating is switched off, then of course a further decrease of the pressure can be observed since the whole product (mixture) cools down. This pressure change might be then referred to as *"pressure after the reaction",* since the reaction is already completed.

In case of method B, the first step and the second step can be performed in two different reaction vessels. Thus, the pressure decrease due to hydrogen consumption can only be observed in the reaction vessel used for the second step. Further, in case of method B it is also possible to include a cooling step between the end of the first step and the beginning of the second step, independently from performing the first and the second step in one reaction vessel or two different reaction vessels. Therefore it is possible to observe a pressure decrease between the first and the second step due to temperature decrease.

The term *"reaction time"* within the context of the present application depends on the manner on how the methods according to the invention are performed

In case the methods according to the invention are performed in a batchwise manner in a closed reaction vessel, the reaction time is the time range starting when the heating of the reaction mixture is started, respectively switched on and the point in time when a constant value of the pressure during the reaction is observed for the first time, i.e. the reaction is completed. If a constant pressure value is observed, the reaction can either be immediately stopped or can be allowed to rest at the reaction conditions for some further time. This time, however, then also accounts to the reaction time. In particular, the reaction time can be at least 1 minute, or at least 5 minutes, or at least 10 minutes, or at least 15 minutes, or at least 20 minutes, or at least 25 minutes, or at least 30 minutes, or at least 35 minutes, or at least 40 minutes, or at least 45 minutes, or at least 50 minutes, or at least 55 minutes, or at least 60 minutes, or at least 65 minutes, or at least 70 minutes or at least 75 minutes, or at least 80 minutes, or at least 85 minutes, or at least 90 minutes, or at least 120 minutes, or at least 180 minutes. Preferably the reaction time is at least 1 minute and up to 90 minutes.

In case method A according to the invention is performed in a fed-batch manner the reaction time is the time range starting from the point in time when the feeding of the last educt, e.g. the at least one primary/secondary amine or the at least one reducing sugar, or hydrogen, is stopped and the point in time when a constant value of the pressure during the reaction is observed, i.e. the reaction is completed.

In case method B according to the invention is performed in a fed-batch manner the reaction time of the first step is the time starting from the point in time when feeding of the last educt, e.g. the at least one primary/secondary amine or the at least one reducing sugar, is stopped and the point in time that the first step is terminated. Termination of the first step can occur by cooling the reaction mixture, evacuating the reactor mixture from the vessel, e.g. to intermediate storage, intermediate purification or transfer to second vessel to perform second step, or when the second step is initiated in the first vessel, e.g. by supplying hydrogen to the vessel. The reaction time of the second step is the time range starting from the point in time when the feeding of hydrogen is stopped and the point in time when a constant value of the pressure during the reaction is observed, i.e. the reaction is completed.

In particular, the reaction time can be at least 1 second, or at least 30 seconds, or at least 1 minute, or at least 5 minutes, or at least 10 minutes, or at least 15 minutes, or at least 20 minutes, or at least 25 minutes, or at least 30 minutes, or at least 35 minutes, or at least 40 minutes, or at least 45 minutes, or at least 50 minutes, or at least 55 minutes, or at least 60 minutes, or at least 65 minutes, or at least 70 minutes or at least 75 minutes, or at least 80 minutes, or at least 85 minutes, or at least 90 minutes, or at least 120 minutes, or at least 180 minutes and up to 5 hours. Preferably the reaction time is at least 1 minute and up to 90 minutes.

In case the methods according to the invention are performed in a continuous manner, the reaction time can be regarded as the average residence time of the reaction mixture within the reaction vessel, since there is an ongoing and continuous supply of the educts on the one hand, and an ongoing and continuous removal of the obtained reaction product (mixture) on the other hand. In particular, the reaction time can be at least 1 second, or at least 30 seconds, or at least 1 minute, or at least 5 minutes, or at least 10 minutes, or at least 15 minutes, or at least 20 minutes, or at least 25 minutes, or at least 30 minutes, or at least 35 minutes, or at least 40 minutes, or at least 45 minutes, or at least 50 minutes, or at least 55 minutes, or at least 60 minutes, or at least 65 minutes, or at least 70 minutes or at least 75 minutes, or at least 80 minutes, or at least 85 minutes, or at least 90 minutes, or at least 120 minutes, or at least 180 minutes. Preferably the reaction time is at least 1 minute and up to 60 minutes.

Both methods according to the invention can be performed in a batchwise manner, or in a fed-batch manner or on a continuous manner.

The term *"batchwise manner",* respectively *"batchwise"* within the context of the present application is commonly known by a skilled person and means that at least some necessary educts, like reducing sugar are loaded into the reaction vessel prior to the actual reaction taking place, the reaction vessel is then closed and pressurized. The pressurization of the reaction vessel can be achieved by e.g. loading the closed reaction vessel with hydrogen and/or nitrogen until the desired reaction pressure is achieved. Only after the reaction is completed, the product (mixture) is discharged from the reaction vessel and the reaction vessel can be either cleaned if necessary, or filled again with educts.

In case of method B according to the invention the first step and the second step can also performed in the same reaction vessel or in two different reaction vessels. The (intermediate) product mixture obtained in the first step can be either transferred into a second reaction vessel in order to undergo the second step there or it can remain in the same reaction vessel and the reaction conditions (e.g. supply of hydrogen) are changed to those of the second step.

Further, in case of method B it is also possible to include a cooling step and/or a compound removing step, like a degassing step, between the end of the first step and the beginning of the second step, independently from performing the first and the second step in one reaction vessel or two different reaction vessels

Suitable reaction vessels for performing the methods according to the invention in a batchwise manner are for example stirred tank reactors, autoclaves, loop reactors or gas lift reactors.

The term *"fed-batch manner",* respectively *"fed-batch"* within the context of the present application is commonly known by the skilled person and means that the reaction vessel is at least partly filled with educts at the start; or at least filled with one of the educts. The reactor is then closed and heating is started. Then, if the reaction temperature set point is reached, further educt(s) (either more of those that were initially present, or new ones) are supplied (also called "*fed*") into the reaction vessel until e.g. the maximum filling capacity of the reaction vessel is achieved, or the maximum duration of the experiment (e.g. end of the working day) is achieved. In a fed batch run, no reaction mixture or product mixture is removed from the reactor during the run. Only after the reaction is completed, the product (mixture) is discharged from the reaction vessel and the reaction vessel can be either cleaned if necessary, or filled again with educts. The reaction pressure is preferably kept constant e.g. by means of a pressure regulator, if the methods according to the invention is performed in fed-batch manner.

Suitable reaction vessels for performing the methods according to the invention in a fed-batch manner are for example stirred tank reactors, stirred autoclaves, loop reactors or gas lift reactors.

In case of method B according to the invention, the first step can be performed in in batch mode and the second step can be performed in fed batch mode by e.g. supplying hydrogen to the reaction mixture. It is also possible that in case of method B according to the invention the first step is performed in fed batch mode and the second step in batch mode.

The term *"continuous manner"* within the context of the present application is also commonly known by the skilled person and means that the methods according to the invention are conducted continuously. The educts are constantly fed into the reaction vessel, and products, respectively the reaction mixture are constantly withdrawn from the reactor. During their residence time within the reaction vessel, the educts react with each other. The supply of educts and the discharge of products take place at the same time within the reaction vessel. Supply of the educts and discharge of the products can occur at the same location (i.e. in the same uniform mixing zone), but it is also possible to supply educts, e.g. reducing sugar, hydrogen and primary amine and/or secondary amine into the reaction vessel at one end of the reaction vessel. This reaction mixture is then allowed to pass through a fixed bed of supported hydrogenation catalyst. Thus, the educts react with each other under the formation of the product (mixture). The resulting product (mixture) is then discharged from the reaction vessel at another end of the reaction vessel.

In case of method B according to the invention, the first step and the second step are performed in two separate reaction vessels. In each of the reaction vessels the respective reaction conditions for the two different steps are prevailing. Educts like reducing sugar and optionally solvent can be added in front of the first reaction vessel and/or in front of the second reaction vessel. The final product, respectively product mixture can be withdrawn from the second reaction vessel. It is also possible to withdraw the reaction mixture after the reaction mixture has passed through the first reaction vessel for example in order recycle, or purify the reaction mixture or for heat exchange purposes.

Suitable reaction vessels for performing the methods according to the invention in a continuous manner are for example stirred tank reactors, stirred autoclaves, loop reactors, tubular reactors (with or without a fixed bed of catalyst) or gas lift reactors.

After the reaction of the at least one reducing sugar, hydrogen and the at least one primary amine and/or at least one secondary amine is completed, a reaction effluent is obtained. The reaction effluent contains at least one acyclic N-substituted ethylene amine as desired product (mixture). The reaction effluent can further contain additional solvent, residues of educts, co-products (e.g. co-produced water), by-products, supported hydrogenation catalyst or mixtures thereof.

Depending on the composition of the reaction effluent, the reaction effluent can be either purified or can be processed further directly without any purification.

In case solid impurities, e.g. supported hydrogenation catalyst are present in the reaction effluent, then these impurities can be removed e.g. by filtration, centrifugation or settlement and thus the reaction effluent is purified. Such a solid separation system can also be built into the reactor, prohibiting supported hydrogenation catalyst particles to leave the reactor with the effluent stream.

In case liquid, respectively dissolved impurities are present in the reaction effluent, e.g. by-products, then these impurities can be removed e.g. by distillation, decantation, pervaporation, ultrafiltration or other suitable separation methods in order to purify the reaction effluent.

In general, the purification of the reaction effluent is not a mandatorily necessary measure. The reaction effluent can be analyzed e.g. by gas-chromatographic analysis and depending on the outcome of the analysis it can be decided if further purification is necessary.

The at least one *N*-substituted acyclic ethylenediamine obtained by the methods according to the invention can be used as building block for surfactants and fabric softener, as epoxy curing agent, as catalyst for manufacturing polyurethane, or as ligand for metals.

The at least one *N*-substituted acyclic ethylenediamine obtained by the methods according to the invention can be directly used as building block for surfactants and fabric softener, as epoxy curing agent, as catalyst for manufacturing polyurethane, or as ligand for metals without any further purification.

The at least one *N*-substituted acyclic ethylenediamine obtained by the method according to the invention can be also first purified and then used as building block for surfactants and fabric softener, as epoxy curing agent, as catalyst for manufacturing polyurethane, as ligand for metals.

### EXAMPLES

The examples are to be understood as illustrating the method according to the invention. Examples 1 to 14 and Comparative Examples 1 to 11 illustrate method A according to the invention. Example 15 illustrates method B according to the invention. The examples are however not to be construed as limiting the scope of the invention.

### Comparison of effect of supported hydrogenation catalysts vs. unsupported hydrogenation catalysts on yield of acyclic, N-substituted ethylene diamine

### Examples 1 to 5

Examples 1 to 5 are carried out using 5 gram (0.028 mol) of glucose as the reducing sugar and 25 gram of 60wt% aqueous dimethylamine (DMA) as secondary amine. No additional solvent is added to the reaction mixture. The amount of supported hydrogenation catalyst added to the reaction mixture is the same (0.5 gram) for examples 1 to 5. The specific supported hydrogenation catalyst is varied in Example 1 to 5 as follows:

| **Example** | **Supported hydrogenation catalyst** | **Metal content of catalyst active metal center of supported hydrogenation catalyst [wt%]** | **Support substrate** |
|---|---|---|---|
| 1 | Ru/C | 5 wt% Ru | carbon |
| 2 | Ni-6458P | 56 wt% Ni | SiO₂ and small amount of Al₂O₃ |
| 3 | Ni-3354E | 60 wt% Ni promoted with Mo | SiO₂ |
| 4 | Ni-5249P | 64 wt% Ni | SiO₂ |
| 5 | Ni/SiO₂-Al₂O₃ | 65 wt% Ni | SiO₂-Al₂O₃ |

Ni-6458P, Ni-5249P, Ni-3354E can be obtained from BASF. Ni/SiO₂-Al₂O₃ (65 wt% Ni) can be obtained from Sigma-Aldrich. Ru/C can be obtained from Sigma Aldrich.

The reducing sugar, the supported hydrogenation catalyst and the secondary amine are loaded into a 50 mL stainless steel Parr reactor (Parr Series No 32092D - 50mL) equipped with electric heating. The reaction mixture is then pressurized with hydrogen at room temperature up to a reaction pressure of 75 bar. The pressure was measured by means of an analogous manometer mounted on the top of the reactor lid. The reaction mixture is then heated to a reaction temperature of 125°C while stirring.

During heating, the pressure increases up to a maximum value of 94 bar within 13 minutes.

Then the pressure starts to decrease until a value of 75 bar and is then kept constant by addition of more hydrogen.. The reaction time is 60 minutes.

The supported hydrogenation catalyst is then removed from the reaction effluent by filtration.

The obtained reaction effluent is analyzed using gas-chromatographic analysis (gas chromatograph: Agilent 6890N; GC column: Varian capillary column CP-WAX for volatile amines and diamines 25m-0.32mm-1,2µm #CP7422).

The composition of the reaction effluent is listed in Table 1:

**Table 1:**

| **Example** | **Supported hydrogenation catalyst** | **Acyclic *N-*substituted ethylene diamine** | **Yield of Acyclic *N-*substituted ethylene diamine [C% based on amount of reducing sugar]** | **by-products** |
|---|---|---|---|---|
| **1** | Ru/C | N,N,N',N'-tetramethyl ethylenediamine (TMEDA) | 50.6 | N,N-dimethyl aminoethanol (DMAE); N,N,N'N'-tetramethyl propylenediamine (TMPDA); N,N-dimethylamino-2-propanol (DMA-2-PropOH); N,N-dimethylamino-2-propanone (DMA-2-Propanone), N,N-dimethyl formamide (DMF) |
| **2** | Ni-6458P | N,N,N',N'-tetramethyl ethylenediamine (TMEDA) | 47.5 | See Ex. 1 |
| **3** | Ni-3354E | N,N,N',N'-tetramethyl ethylenediamine (TMEDA) | 43.9 | See Ex. 1 |
| **4** | Ni-5249P | N,N,N',N'-tetramethyl ethylenediamine (TMEDA) | 41.5 | See Ex. 1 |
| **5** | Ni/SiO₂-Al₂O₃ | N,N,N',N'-tetramethyl ethylenediamine (TMEDA) | 46.8 | See Ex. 1 |

### Comparative Examples 1 to 3

Comparative Examples 1 to 3 are conducted in the same way as Examples 1 to 5 with the sole difference that in Comparative Examples 1 to 3 Raney-nickel catalysts are used instead of supported hydrogenation catalysts. The specific Raney-nickel catalyst is varied in Comparative Examples 1 to 3 as follows:

| **Comparative Example** | **Raney-nickel catalyst** | **Ni content [wt%]** | **Avg particle size** | **Al content [wt%]** | **Fe content [wt%]** |
|---|---|---|---|---|---|
| 1 | Raney® Ni-4200 | >93 wt% | 20-50µm | <6.5 wt% | <0.8 wt% |
| 2 | Raney® Ni-5601 | >89 wt% | 15-45µm | 4-10 wt% | <0.7 wt% |
| 3 | Raney® Ni-6800 | >94.8 wt% | 10-15.5µm | 3.4-5 wt% | <0.5 wt% |

Raney® Ni-4200, Raney® Ni-5601, Raney® Ni-6800 can be obtained from Grace.

The obtained reaction effluent is analyzed using gas-chromatographic analysis (gas chromatograph: Agilent 6890N; GC column: Varian capillary column CP-WAX for volatile amines and diamines 25m-0.32mm-1.2µm #CP7422).

The composition of the reaction effluent is listed in Table 2:

**Table 2:**

| **Comparative Example** | **Raney-nickel catalyst** | **Acyclic *N-*substituted ethylene diamine** | **Yield of Acyclic *N-*substituted ethylene diamine [C% based on amount of reducing sugar]** | **by-products** |
|---|---|---|---|---|
| **1** | Raney® Ni-4200 | N,N,N',N'-tetramethyl ethylenediamine (TMEDA) | 19.1 | N,N-dimethyl aminoethanol (DMAE); N,N,N'N'-tetramethyl propylenediamine (TMPDA); N,N-dimethylamino-2-propanol (DMA-2-PropOH); N,N-dimethylamino-2-propanone (DMA-2-Propanone), N,N-dimethyl formamide (DMF) |
| **2** | Raney® Ni-5601 | N,N,N',N'-tetramethyl ethylenediamine (TMEDA) | 22.8 | See Comp. Ex. 1 |
| **3** | Raney® Ni-6800 | N,N,N',N'-tetramethyl ethylenediamine (TMEDA) | 22.5 | See Comp. Ex. 1 |

Examples 1 to 5 clearly result in higher yields of TMEDA compared to Comparative Examples 1 to 3. Thus it is demonstrated that the presence of supported hydrogenation catalyst influences the yield of TMEDA and minimizes the formation of by-products.

### Comparison of effect of supported hydrogenation catalysts vs. unsupported hydrogenation catalysts with and without addition of a support substrate on yield of acyclic. N-substituted ethylene diamine

### Example 6

Example 6 is conducted in the same way as Examples 1 to 5 with the sole difference that no commercially available supported hydrogenation catalyst is used, but instead the supported hydrogenation catalyst is manufactured as follows:

A nickel containing supported hydrogenation catalyst is prepared through incipient wetness of an aqueous solution of nickel nitrate hexahydrate to a SiO₂ (Aerosil 380) support, followed by activation with H₂ at 500°C (1 hour). Thus, a Ni/SiO₂ (12wt% Ni) catalyst is obtained.

The obtained reaction effluent is analyzed using gas-chromatographic analysis (gas chromatograph: Agilent 6890N; GC column: Varian capillary column CP-WAX for volatile amines and diamines 25m-0.32mm-1.2µm #CP7422).

The composition of the reaction effluent is listed in Table 3:

**Table 3:**

| **Example** | **Acyclic *N-*substituted ethylene diamine** | **Yield of Acyclic *N*-substituted ethylene diamine [C% based on amount of reducing sugar]** | **by-products** |
|---|---|---|---|
| **6** | N,N,N',N'-tetramethyl ethylenediamine (TMEDA) | 41.3 | N,N-dimethyl aminoethanol (DMAE); N,N,N'N'-tetramethyl propylenediamine (TMPDA); N,N-dimethylamino-2-propanol (DMA-2-PropOH); N,N-dimethylamino-2-propanone (DMA-2-Propanone), N,N-dimethyl formamide (DMF) |

### Comparative Examples 4 and 5

Comparative Examples 4 and 5 are conducted in the same way as Examples 1 to 5 with the sole difference that in Comparative Example 4 Raney Ni-5601 is used as unsupported hydrogenation catalyst without the addition of any support substrate, while in Comparative Example 5 Raney Ni-5601 is used as unsupported hydrogenation catalyst together with 0.5 gram of SiO₂ (Aerosil 380) as support substrate which is additionally added to the reaction mixture.

The obtained reaction effluent is analyzed using gas-chromatographic analysis (gas chromatograph: Agilent 6890N; GC column: Varian capillary column CP-WAX for volatile amines and diamines 25m-0.32mm-1.2µm #CP7422).

The composition of the reaction effluent is listed in Table 4:

**Table 4:**

| **Comparative Example** | **Raney-nickel catalyst** | **Acyclic *N-*substituted ethylene diamine** | **Yield of Acyclic *N-*substituted ethylene diamine [C% based on amount of reducing sugar]** | **by-products** |
|---|---|---|---|---|
| **4** | Raney® Ni-5601 | N,N,N',N'-tetramethyl ethylenediamine (TMEDA) | 22.8 | N,N-dimethyl aminoethanol (DMAE); N,N,N'N'-tetramethyl propylenediamine (TMPDA); N,N-dimethylamino-2-propanol (DMA-2-PropOH); N,N-dimethylamino-2-propanone-(DMA-2-Propanone), N,N-dimethyl formamide (DMF) |
| **5** | Raney® Ni-5601 + 0.5 gram of SiO₂ | N,N,N',N'-tetramethyl ethylenediamine (TMEDA) | 26.2 | See Comp. Ex. 4 |

Example 6 clearly results in higher yields of TMEDA compared to Comparative Examples 4 and 5. Thus, it is demonstrated that the presence of supported hydrogenation catalyst influences the yield of TMEDA.

### Comparison of effect of using reducing sugar on formation of N-substituted, acyclic ethylene diamine

### Examples 7 to 9

Example 7 is conducted the same way as Example 1. Examples 8 and 9 are also conducted the same way as Example 1 with the sole difference that in Example 8, 2.5 gram (0.0073 mol) of cellobiose as the reducing sugar instead of glucose is used and in Example 9, 2.5 gram (0.0073 mol) of maltose as the reducing sugar instead of glucose is used. Thus, the reducing sugar is varied in Examples 7 to 9 as follows:

| **Example** | **Reducing sugar** |
|---|---|
| 7 | Glucose |
| 8 | Cellobiose |
| 9 | Maltose |

The obtained reaction effluent is analyzed using gas-chromatographic analysis (gas chromatograph: Agilent 6890N; GC column: Varian capillary column CP-WAX for volatile amines and diamines 25m-0.32mm-1.2µm #CP7422).

The composition of the reaction effluent is listed in Table 5:

**Table 5:**

| **Example** | **Acyclic *N-*substituted ethylene diamine** | **Yield of Acyclic *N*-substituted ethylene diamine [C% based on amount of reducing sugar]** | **by-products** |
|---|---|---|---|
| **7** | N,N,N',N'-tetramethyl ethylenediamine (TMEDA) | 50.6 | N,N-dimethyl aminoethanol (DMAE); N,N,N'N'-tetramethyl propylenediamine (TMPDA); N,N-dimethylamino-2-propanol (DMA-2-PropOH); N,N-dimethylamino-2-propanone (DMA-2-Propanone), N,N-dimethyl formamide (DMF) |
| **8** | N,N,N'N'-tetramethyl ethylenediamine (TMEDA) | 33.1 | N,N-dimethyl aminoethanol (DMAE); N,N,N'N'-tetramethyl propylenediamine (TMPDA); N,N-dimethylamino-2-propanol (DMA-2-PropOH); N,N-dimethyl formamide (DMF) |
| **9** | N,N,N',N'-tetramethyl ethylenediamine (TMEDA) | 34.1 | See Ex. 8 |

### Comparative Example 6

Comparative Example 6 is conducted the same way as Example 1 with the sole differences that 3 gram (0.0088 mol) of sucrose as non-reducing sugar instead of reducing sugar glucose and that the reaction time was 90 minutes.

The obtained reaction effluent is analyzed using gas-chromatographic analysis (gas chromatograph: Agilent 6890N; GC column: Varian capillary column CP-WAX for volatile amines and diamines 25m-0.32mm-1.2µm #CP7422).

The composition of the reaction effluent is listed in Table 6:

**Table 6:**

| **Comparative Example** | **Acyclic *N-*substituted ethylene diamine** | **Yield of Acyclic *N-*substituted ethylene diamine [C% based on amount of reducing sugar]** | **by-products** |
|---|---|---|---|
| **6** | N,N,N',N'-tetramethyl ethylenediamine (TMEDA) | 0.0 | N,N-dimethyl formamide (DMF); sucrose |

Examples 7 to 9 clearly results in higher yields of TMEDA compared to Comparative Example 6. The fact, that in Comparative Example 6 no conversion of sucrose into TMEDA could have been observed demonstrates the need of reducing sugars as substrates in order to benefit from the low temperatures as used in the methods according to the invention and further in order to influence the yield of TMEDA positively.

### Comparative Example 7

Comparative Example 7 is conducted the same way as Example 1 and Comparative example 6 with the sole differences that 3 gram (0.0088 mol) of sucrose is used instead of glucose and that the reaction temperature is 220°C instead of 125°C and further that the reaction time was 120 minutes.

The obtained reaction effluent is analyzed using gas-chromatographic analysis (gas chromatograph: Agilent 6890N; GC column: Varian capillary column CP-WAX for volatile amines and diamines 25m-0.32mm-1.2µm #CP7422).

The composition of the reaction effluent is listed in Table 7:

**Table 7:**

| **Comparative Example** | **Acyclic *N-*substituted ethylene diamine** | **Yield of Acyclic *N*-substituted ethylene diamine [C% based on amount of reducing sugar]** | **by-products** |
|---|---|---|---|
| **7** | N,N,N',N'-tetramethyl ethylenediamine (TMEDA) | 6.3 | N,N-dimethyl aminoethanol (DMAE); 1,4-dimethyl piperazine (also with methyl substituents); N,N-dimethyl formamide (DMF);; N,N-dimethylacetamide, N-methylpyrrole (also with methyl substituents), pyrazine (also with methyl substituents) |
| | N,N,N'-trimethyl ethylenediamine (TriMEDA) | 6.1 | |

Comparative Example 7 clearly demonstrates that the conversion of non-reducing sugars like sucrose need comparatively vigorous reaction conditions, in particular higher reaction temperatures in order to obtain an acyclic *N*-substituted ethylene diamine (here: TMEDA) at all. However, the yield and purity of the thus obtained acyclic *N*-substituted ethylene diamine (here: TMEDA) is bad. The high temperatures are leading the catalyst towards disproportionation reactions, in which methyl groups are transferred. TriMEDA is the result of disproportionation reactions. Further, also the cyclization reactions can be observed. Pyrazine, N-methylpyrrole or piperazine are the results of such cyclization reactions.

### Further Examples varying educts, reaction conditions or educt ratios

### Examples 10 to 14

Example 10 is carried out using 1 gram (0.0056 mol) of glucose as the reducing sugar and 27 gram of 2M dimethylamine (DMA) solution in methanol as secondary amine. No additional solvent is added to the reaction mixture. The amount of supported hydrogenation catalyst Ni-6458P added to the reaction mixture is 0.3 gram. The reaction is then further carried out as described in Examples 1 to 5.
Example 11 is carried out using 2.5 gram (0.014 mol) of glucose as the reducing sugar and 25 gram of 50wt% aqueous monomethylamine (MMA) as primary amine. No additional solvent is added to the reaction mixture. The amount of supported hydrogenation catalyst Ni-6458P added to the reaction mixture is 0.5 gram. The reaction is then further carried out as described in Examples 1 to 5.
Example 12 is carried out using 2.3 gram (0.013 mol) of glucose as the reducing sugar and 32 gram of ethanolamine as primary amine. No additional solvent is added to the reaction mixture. The amount of supported hydrogenation catalyst Ni-6458P added to the reaction mixture is 0.4 gram. The reaction is then further carried out as described in Examples 1 to 5 with the sole differences that the reaction temperature is 130°C and that the reaction time is 120 minutes.
Example 13 is carried out using 1.5 gram (0.008 mol) of glucose as the reducing sugar and 33 gram of N-methyl ethanolamine as secondary amine. No additional solvent is added to the reaction mixture. The amount of supported hydrogenation catalyst Ni-6458P added to the reaction mixture is 0.4 gram. The reaction is then further carried out as described in Examples 1 to 5 with the sole differences that the reaction temperature is 130°C and that the reaction time is 120 minutes.
Example 14 is carried out using 2 gram of xylose (0.013 mol) as the reducing sugar and 33 gram of N-methyl ethanolamine as secondary amine. No additional solvent is added to the reaction mixture. The amount of supported hydrogenation catalyst Ni-6458P added to the reaction mixture is 0.4 gram. The reaction is then further carried out as described in Examples 1 to 5 with the sole differences that the reaction temperature is 130°C and that the reaction time is 120 minutes.

The obtained reaction effluents are analyzed using gas-chromatographic analysis (GC column: 25m CP WAX). In case of Examples 12 to 14 also ¹³C-Nuclear Magnetic Resonance (NMR) spectroscopy analysis was used. The composition of the reaction effluent is listed in Table 8:

**Table 8:**

| **Example** | **Acyclic *N*-substituted ethylene diamine** | **Yield of Acyclic *N-*substituted ethylene diamine [C% based on amount of reducing sugar]** | **by-products** |
|---|---|---|---|
| **10** | N'N,N',N'-tetramethyl ethylenediamine (TMEDA) | 66.3 | N,N-dimethyl aminoethanol (DMAE); N,N,N'N'-tetramethyl propylenediamine (TMPDA); N,N-dimethylamino-2-propanol (DMA-2-PropOH); N,N-dimethylamino-2-propanone (DMA-2-Propanone), N,N-dimethyl formamide (DMF) |
| **11** | N,N'-dimethyl ethylenediamine (DMEDA) | 42.6 | N-methyl aminoethanol (MAE), 3-methylamino-1,2 propanediol; 1,4-dimethyl piperazine (1,4-DMPIP) |
| **12** | N,N'-bis(2-hydroxyethyl)ethylenediamine (BHEEDA) | 39.7 | N-methyl aminoethanol (MAE) |
| **13** | N,N'-bis(2-hydroxyethyl)-N,N'-dimethylethylenediamine (BHEDMEDA) | 81.9 | methyl diethanolamine (MDEA); N,N-dimethyl aminoethanol (DMAE) |
| **14** | N,N'-bis(2-hydroxyethyl)-N,N'-dimethylethylenediamine (BHEDMEDA) | 57.9 | methyl diethanolamine (MDEA); N,N-dimethyl aminoethanol (DMAE) |

In Example 11 only little cyclization to piperazines is observed. This is a result of the particularly mild conditions that can be used according to the present invention.

### Comparative Examples 8 to 11

Comparative Example 8 is conducted as Example 10 with the sole difference that Raney® Ni-5601 is used.
Comparative Example 9 is conducted as Example 11 with the sole difference that Raney® Ni-5601 is used.
Comparative Example 10 is conducted as Example 12 with the sole difference that Raney® Ni-5601 is used.
Comparative Example 11 is conducted as Example 13 with the sole difference that Raney® Ni-5601 is used.

The obtained reaction effluents are analyzed using gas-chromatographic analysis (gas chromatograph: Agilent 6890N; GC column: Varian capillary column CP-WAX for volatile amines and diamines 25m-0.32mm-1.2µm #CP7422). In case of Comparative Examples 8 and 9 also ¹³C-Nuclear Magnetic Resonance (NMR) spectroscopy analysis was used.

The composition of the reaction effluent is listed in Table 9:

**Table 9:**

| **Comparative Example** | **Acyclic *N*-substituted ethylene diamine** | **Yield of Acyclic *N-*substituted ethylene diamine [C% based on amount of reducing sugar]** | **by-products** |
|---|---|---|---|
| **8** | N,N,N',N'-tetramethyl ethylenediamine (TMEDA) | 36 | N,N-dimethyl aminoethanol (DMAE); N,N,N'N'-tetramethyl propylenediamine (TMPDA); N,N-dimethylamino-2-propanol (DMA-2-PropOH); N,N-dimethylamino-2-propanone (DMA-2-Propanone), N,N-dimethyl formamide (DMF) |
| **9** | N,N'-dimethyl ethylenediamine (DMEDA) | 19.9 | N-methyl aminoethanol (MAE), 1,4-dimethyl piperazine (1,4-DMPIP) |
| **10** | N,N'-bis(2-hydroxyethyl)ethylenediamine (BHEEDA) | 24.0 | N-methyl aminoethanol (MAE) |
| **11** | N,N'-bis(2-hydroxyethyl)-N,N'-dimethylethylenediamine (BHEDMEDA) | 60.5 | methyl diethanolamine (MDEA); N,N-dimethyl aminoethanol (DMAE) |

Examples 10 to 14 in comparison with Comparative Example 8 to 11 also demonstrate the advantages of the method according to the invention.

### Example for 2-step process

### Example 15

Example 15 is carried out using 100 ml of an aqueous glucose solution (500 g/l, 50 g glucose, 0.28 mol) as reducing sugar, 300 g of N-methylethanolamine (MMEA, 4 mol) as secondary amine and 5 g of supported hydrogenation catalyst (KL6504K-P)(supplier: CRI, powder, 71 wt% Ni, support silica). No additional solvent is added to the reaction mixture. The experiment is performed via a 2-step process. In a first step, MMEA and hydrogenation catalyst are loaded in a 1 l Parr reactor equipped with electrical heating. The reaction mixture is pressurized at room temperature with 3 bar nitrogen. The pressure is monitored by means of a digital manometer mounted on top of the reactor lid. Subsequently, the reaction mixture is heated to 130°C while stirring the reaction mixture. During the heating, the pressure increases up to a maximum value of 5.7 bar. Subsequently, the glucose solution is added in fed batch mode (5 ml/min). After finishing the addition of the glucose solution, the reaction mixture is stirred for an additional 30 min at the reaction temperature of 130°C. During the addition of the glucose solution and post reaction, the pressure increased to 8.5 bar, mainly due to a decrease of the headspace in the reactor. Subsequently, nitrogen is vented at 130°C and the reaction mixture is pressurized with 75 bar H₂ at the reaction temperature of 130°C. After adding hydrogen, the reaction mixture is stirred for 120 min at 130°C. During the post reaction, the pressure stabilized at a constant value. The reaction effluent is analyzed using gas-chromatographic analysis (gas chromatograph: Agilent GC 7890A; GC column: RTX volamine 60 m x 0.32 mm x 5 µm).

The composition of the reaction effluent is listed in Table 10.

**Table 10**

| **Example** | **Supported hydrogenation catalyst** | **Acyclic *N*-substituted ethylene diamine** | **Yield of acyclic *N-*substituted ethylene diamine [C% based on amount of reducing sugar]** | **By-products** |
|---|---|---|---|---|
| **15** | KL6504K-P | N, N'-bis(hyd roxyethyl)-N,N'-dimethylethylenediamine (BHEDMEDA) | 64.0 | N,N-dimethylaminoethanol (DMAE); N,N-dimethyl formamide (DMF) |

## Claims

1. Method for producing at least one *N*-substituted acyclic ethylenediamine, comprising
- reacting at least one reducing sugar, hydrogen and at least one primary amine NH₂R or at least one secondary amine NHR'R" or a mixture of at least one primary amine NH₂R and at least one secondary amine NHR'R"
- in the presence of a supported hydrogenation catalyst
- at a reaction temperature of 50°C to 200 °C, and
- at a reaction pressure being superatmospheric pressure, and wherein
- the molar ratio of the at least one primary amine and/or secondary amine : reducing sugar is at least 6:1, wherein residue R is selected from linear or branched alkyl groups, linear or branched alkanol groups, cyclic alkyl groups which may be substituted and aromatic groups, which may be substituted, wherein residues R' and R" can be identical or different from each other and can each be independently selected from linear or branched alkyl groups, linear or branched alkanol groups, cyclic alkyl groups which may be substituted and aromatic groups which may be substituted, or wherein R' and R" can form together with the nitrogen atom to which the residues R' and R" are attached a heterocyclic alkyl group or a heterocyclic aromatic group.

2. Method for producing at least one N-substituted acyclic ethylene diamine, comprising the steps of
(1) reacting at least one reducing sugar and at least one primary amine NH₂R or at least one secondary amine NH₂R'R" or a mixture of at least one primary amine NH₂R and the at least one secondary NHR'R" in a first step, and
(2) reacting the reaction mixture obtained in step (1) with hydrogen in the presence of a supported hydrogenation catalyst in a second step, wherein
the molar ratio of the at least one primary amine and/or secondary amine : reducing sugar is at least 6:1, wherein residue R is selected from linear or branched alkyl groups, linear or branched alkanol groups, cyclic alkyl groups which may be substituted and aromatic groups, which may be substituted, wherein residues R' and R" can be identical or different from each other and can each be independently selected from linear or branched alkyl groups, linear or branched alkanol groups, cyclic alkyl groups which may be substituted and aromatic groups which may be substituted, or wherein R' and R" can form together with the nitrogen atom to which the residues R' and R" are attached a heterocyclic alkyl group or a heterocyclic aromatic group.

3. Method according to claim 1 or 2, with the provision that no unsupported hydrogenation catalyst is used, in particular no spongy hydrogenation catalyst or no hydrogenation catalyst of the Raney-type.

4. Method according to at least one of the preceding claims, wherein the supported hydrogenation catalyst comprises a metal, in particular copper, nickel, cobalt, iron, ruthenium, platinum, palladium, and two or more thereof.

5. Method according to at least one of the preceding claims, wherein the supported hydrogenation catalyst comprises a support substrate for the catalytic active metal selected from carbon, a polymer or a metal oxide.

6. Method according to at least one of the preceding claims, wherein the residue R of the at least one primary amine or the residues R' and R" of at least one secondary amine are the same or different and each independently selected from hydrogen, linear or branched alkyl groups, linear or branched hydroxyalkyl groups, cyclic alkyl groups, which may be substituted and aromatic groups which may be substituted.

7. Method according to at least one of the preceding claims, with the provision that no ammonia (NH₃) is used as educt for the production of *N*-substituted acyclic ethylene diamines.

8. Method according to at least one of the preceding claims, wherein the at least one reducing sugar is selected from a monosaccharide, a disaccharide, an oligosaccharide or a polysaccharide, and a mixture of two or more thereof.

9. Method according to at least one of the preceding claims, wherein at most 10 C% of heterocyclic by-products are formed based on the total amount of reducing sugar used as educt.

10. Method according to at least one of the preceding claims, wherein the at least one primary amine and/or the at least one secondary amine can be used in form of a solution, wherein the at least one primary amine and/or the at least one secondary amine is dissolved in an aprotic solvent or a protic solvent, in particular water, or methanol, or DMF, or dimethylacetamide.

11. Method according to at least one of the preceding claims, wherein at least one primary amine and/or the at least one secondary amine is used in gaseous form.

12. Method according to at least one of the preceding claims, wherein the reducing sugar is used in solid form or in the form of a solution, wherein the reducing sugar is dissolved in water, alcohols, ethers or dipolar aprotic solvents.

13. Method according to at least one of the preceding claims, wherein the method is performed in a batchwise manner, or a fed-batch manner or in a continuous manner.

## Patentansprüche

1. Verfahren zur Herstellung von mindestens einem *N*-substituierten acyclischen Ethylendiamin, aufweisend
- Umsetzen von mindestens einem reduzierenden Zucker, Wasserstoff und mindestens einem primären Amin NH₂R oder mindestens einem sekundären Amin NHR'R" oder einer Mischung aus mindestens einem primären Amin NH₂R und mindestens einem sekundären Amin NHR'R"
- in Gegenwart eines geträgerten Hydrierungskatalysators
- bei einer Reaktionstemperatur von 50 °C bis 200 °C, und
- bei einem Reaktionsdruck, der überatmosphärischer Druck ist, und wobei
- das molare Verhältnis des mindestens einen primären Amins und/oder sekundären Amins zu reduzierendem Zucker mindestens 6 : 1 beträgt, wobei der Rest R ausgewählt ist aus linearen oder verzweigten Alkylgruppen, linearen oder verzweigten Alkanolgruppen, cyclischen Alkylgruppen, die substituiert sein können, und aromatischen Gruppen, die substituiert sein können, wobei die Reste R' und R" gleich oder verschieden voneinander sein können und jeweils unabhängig voneinander aus linearen oder verzweigten Alkylgruppen ausgewählt sein können, linearen oder verzweigten Alkanolgruppen, cyclischen Alkylgruppen, die substituiert sein können, und aromatischen Gruppen, die substituiert sein können, oder worin R' und R" zusammen mit dem Stickstoffatom, an das die Reste R' und R" gebunden sind, eine heterocyclische Alkylgruppe oder eine heterocyclische aromatische Gruppe bilden können.

2. Verfahren zur Herstellung mindestens eines *N*-substituierten acyclischen Ethylendiamins, das die folgenden Schritte aufweist
(1) Umsetzen mindestens eines reduzierenden Zuckers und mindestens eines primären Amins NH₂R oder mindestens eines sekundären Amins NH₂R'R" oder einer Mischung aus mindestens einem primären Amin NH₂R und dem mindestens einen sekundären NHR'R" in einem ersten Schritt, und
(2) Umsetzen der in Schritt (1) erhaltenen Reaktionsmischung mit Wasserstoff in Gegenwart eines geträgerten Hydrierungskatalysators in einem zweiten Schritt, wobei
das molare Verhältnis des mindestens einen primären Amins und/oder sekundären Amins zu reduzierendem Zucker mindestens 6 : 1 beträgt, wobei der Rest R ausgewählt ist aus linearen oder verzweigten Alkylgruppen, linearen oder verzweigten Alkanolgruppen, cyclischen Alkylgruppen, die substituiert sein können, und aromatischen Gruppen, die substituiert sein können, wobei die Reste R' und R" identisch oder voneinander verschieden sein können und jeweils unabhängig voneinander ausgewählt sein können aus linearen oder verzweigten Alkylgruppen, linearen oder verzweigten Alkanolgruppen, cyclischen Alkylgruppen, die substituiert sein können, und aromatischen Gruppen, die substituiert sein können, oder wobei R' und R" zusammen mit dem Stickstoffatom, an das die Reste R' und R" gebunden sind, eine heterocyclische Alkylgruppe oder eine heterocyclische aromatische Gruppe bilden können.

3. Verfahren nach Anspruch 1 oder 2 mit der Maßgabe, dass kein trägerloser Hydrierungskatalysator, insbesondere kein schwammartiger Hydrierungskatalysator oder kein Hydrierungskatalysator vom Raney-Typ verwendet wird.

4. Verfahren nach mindestens einem der vorstehenden Ansprüche, wobei der geträgerte Hydrierungskatalysator ein Metall, insbesondere Kupfer, Nickel, Kobalt, Eisen, Ruthenium, Platin, Palladium und zwei oder mehr davon aufweist.

5. Verfahren nach mindestens einem der vorstehenden Ansprüche, wobei der geträgerte Hydrierungskatalysator ein Trägersubstrat für das katalytisch aktive Metall, ausgewählt aus Kohlenstoff, einem Polymer oder einem Metalloxid, aufweist.

6. Verfahren nach mindestens einem der vorstehenden Ansprüche, wobei der Rest R des mindestens einen primären Amins oder die Reste R' und R" mindestens eines sekundären Amins gleich oder verschieden sind und jeweils unabhängig voneinander aus Wasserstoff, linearen oder verzweigten Alkylgruppen, linearen oder verzweigten Hydroxyalkylgruppen, cyclischen Alkylgruppen, die substituiert sein können, und aromatischen Gruppen, die substituiert sein können, ausgewählt sind.

7. Verfahren nach mindestens einem der vorstehenden Ansprüche, mit der Maßgabe, dass kein Ammoniak (NH₃) als Edukt für die Herstellung von N-substituierten acyclischen Ethylendiaminen verwendet wird.

8. Verfahren nach mindestens einem der vorstehenden Ansprüche, wobei der mindestens eine reduzierende Zucker ausgewählt ist aus einem Monosaccharid, einem Disaccharid, einem Oligosaccharid oder einem Polysaccharid und einer Mischung aus zwei oder mehreren davon.

9. Verfahren nach mindestens einem der vorstehenden Ansprüche, wobei höchstens 10 C% heterocyclische Nebenprodukte gebildet werden, bezogen auf die Gesamtmenge des als Edukt verwendeten reduzierenden Zuckers.

10. Verfahren nach mindestens einem der vorstehenden Ansprüche, wobei das mindestens eine primäre Amin und/oder das mindestens eine sekundäre Amin in Form einer Lösung verwendet werden kann, wobei das mindestens eine primäre Amin und/oder das mindestens eine sekundäre Amin in einem aprotischen Lösungsmittel oder einem protischen Lösungsmittel, insbesondere Wasser oder Methanol oder DMF oder Dimethylacetamid, gelöst wird.

11. Verfahren nach mindestens einem der vorstehenden Ansprüche, wobei mindestens ein primäres Amin und/oder das mindestens eine sekundäre Amin in gasförmiger Form verwendet wird.

12. Verfahren nach mindestens einem der vorstehenden Ansprüche, wobei der reduzierende Zucker in fester Form oder in Form einer Lösung verwendet wird, wobei der reduzierende Zucker in Wasser, Alkoholen, Ethern oder dipolaren aprotischen Lösungsmitteln gelöst wird.

13. Verfahren nach mindestens einem der vorstehenden Ansprüche, wobei das Verfahren in einer chargenweisen Art und Weise oder in einer fed-batch Art und Weise oder in einer kontinuierlichen Art und Weise durchgeführt wird.

## Revendications

1. Procédé pour la production d'au moins une éthylènediamine N-substituée acyclique, comprenant
- la mise en réaction d'au moins un sucre réducteur, l'hydrogène et au moins une amine primaire NH₂R ou au moins une amine secondaire NHR'R" ou un mélange d'au moins une amine primaire NH₂R et d'au moins une amine secondaire NHR'R"
- en présence d'un catalyseur d'hydrogénation supporté
- à une température de réaction de 50°C à 200°C, et
- à une pression de réaction étant une pression supérieure à la pression atmosphérique, et dans lequel
- le rapport molaire de l'au moins une amine primaire et/ou amine secondaire au sucre réducteur est d'au moins 6:1, dans lequel le résidu R est choisi parmi des groupes alkyles linaires ou ramifiés, des groupes alcanols linéaires ou ramifiés, des groupes alkyles cycliques qui peuvent être substitués et des groupes aromatiques, qui peuvent être substitués, dans lequel les résidus R' et R" peuvent être identiques ou différents l'un de l'autre et peuvent être chacun indépendamment choisis parmi des groupes alkyles linaires ou ramifiés, des groupes alcanols linéaires ou ramifiés, des groupes alkyles cycliques qui peuvent être substitués et des groupes aromatiques qui peuvent être substitués, ou dans lequel R' et R" peuvent former ensemble avec l'atome d'azote auquel les résidus R' et R" sont fixés un groupe alkyle hétérocyclique ou un groupe aromatique hétérocyclique.

2. Procédé pour la production d'au moins une éthylènediamine N-substituée acyclique, comprenant les étapes de
(1) la mise en réaction d'au moins un sucre réducteur et au moins une amine primaire NH₂R ou au moins une amine secondaire NH₂R'R" ou un mélange d'au moins une amine primaire NH₂R et de l'au moins une amine secondaire NHR'R" dans une première étape, et
(2) la mise en réaction du mélange réactionnel obtenu dans l'étape (1) avec l'hydrogène en présence d'un catalyseur d'hydrogénation supporté dans une deuxième étape, dans lequel
le rapport molaire de l'au moins une amine primaire et/ou amine secondaire au sucre réducteur est d'au moins 6:1, dans lequel le résidu R est choisi parmi des groupes alkyles linaires ou ramifiés, des groupes alcanols linéaires ou ramifiés, des groupes alkyles cycliques qui peuvent être substitués et des groupes aromatiques, qui peuvent être substitués, dans lequel les résidus R' et R" peuvent être identiques ou différents l'un de l'autre et peuvent être chacun indépendamment choisis parmi des groupes alkyles linaires ou ramifiés, des groupes alcanols linéaires ou ramifiés, des groupes alkyles cycliques qui peuvent être substitués et des groupes aromatiques qui peuvent être substitués, ou dans lequel R' et R" peuvent former ensemble avec l'atome d'azote auquel les résidus R' et R" sont fixés un groupe alkyle hétérocyclique ou un groupe aromatique hétérocyclique.

3. Procédé selon la revendication 1 ou 2, à condition qu'aucun catalyseur d'hydrogénation non supporté n'est utilisé, en particulier aucun catalyseur d'hydrogénation poreux ou aucun catalyseur d'hydrogénation du type Raney.

4. Procédé selon au moins l'une des revendications précédentes, dans lequel le catalyseur d'hydrogénation supporté comprend un métal, en particulier cuivre, nickel, cobalt, fer, ruthénium, platine, palladium et deux ou plus de ceux-ci.

5. Procédé selon au moins l'une des revendications précédentes, dans lequel le catalyseur d'hydrogénation supporté comprend un substrat de support pour le métal catalytiquement actif choisi parmi le carbone, un polymère ou un oxyde métallique.

6. Procédé selon au moins l'une des revendications précédentes, dans lequel le résidu R de l'au moins une amine primaire ou les résidus R' et R" d'au moins une amine secondaire sont les mêmes ou différents et chacun indépendamment choisis parmi l'hydrogène, des groupes alkyles linéaires ou ramifiés, des groupes hydroxyalkyles linéaires ou ramifiés, des groupes alkyles cycliques, qui peuvent être substitués et des groupes aromatiques qui peuvent être substitués.

7. Procédé selon au moins l'une des revendications précédentes, à condition que l'ammoniac (NH₃) ne soit pas utilisé comme éduit pour la production d'éthylènediamines N-substituées acycliques.

8. Procédé selon au moins l'une des revendications précédentes, dans lequel l'au moins un sucre réducteur est choisi parmi un monosaccharide, un disaccharide, un oligosaccharide ou un polysaccharide, et un mélange de deux ou plus de ceux-ci.

9. Procédé selon au moins l'une des revendications précédentes, dans lequel au maximum 10 %C de sous-produits hétérocycliques sont formés basé sur la quantité totale de sucre réducteur utilisé comme éduit.

10. Procédé selon au moins l'une des revendications précédentes, dans lequel l'au moins une amine primaire et/ou l'au moins une amine secondaire peut être utilisée sous forme d'une solution, dans lequel l'au moins une amine primaire et/ou l'au moins une amine secondaire est dissoute dans un solvant aprotique ou un solvant protique, en particulier l'eau, ou le méthanol, ou le DMF, ou le diméthylacétamide.

11. Procédé selon au moins l'une des revendications précédentes, dans lequel au moins une amine primaire et/ou l'au moins une amine secondaire est utilisée sous forme gazeuse.

12. Procédé selon au moins l'une des revendications précédentes, dans lequel le sucre réducteur est utilisé sous forme solide ou sous forme d'une solution, dans lequel le sucre réducteur est dissous dans l'eau, alcools, éthers, ou solvants aprotiques dipolaires.

13. Procédé selon au moins l'une des revendications précédentes, dans lequel le procédé est réalisé d'une manière discontinue, ou d'une manière discontinue alimentée ou d'une manière continue.
